# EUROPEAN PATENT APPLICATION

(11) **EP 1 527 772 A1**
(43) Date of publication of application: **04.05.2005**
(21) Application number: 04019514.1
(22) Date of filing: 17.08.2004
(51) Int. Cl.: A61K 9/12, A61K 31/57, A61K 31/573

(54) **An aerosol pharmaceutical solution formulation suitable for oral or nasal inhalation containing glucocorticoids stable to the storage; method for stabilizing formulations and use of a stabilizer**

(30) Priority: 30.10.2003 AR 0303969
(71) Applicant: Laboratorio Pablo Cassara S.r.L., 1408 Buenos Aires (AR)
(72) Inventor: Vega, Julio César, San Martin, 1650 Province of Buenos Aire (AR); De Bonis, Fabian, 1407 City of Buenos Aires (AR)
(74) Representative: Müller, Gerald Christian

(57) **Abstract**

Aerosol pharmaceutical solution formulations containing glucocorticosteroids stabilized by adding water or a mixture of water and citric acid, avoiding corrosion of the elements of container under standard storage conditions are described. The formulations comprise:
- between 0.05 and 1.0 % by weight of a glucocorticoid having a C-20 ketone and OH group in carbons 17 and/or 21 as active substance;
- between 0.10 and 3 % by weight of a selected stabilizer selected between water, or a mixture of water and organic acid selected between citric acid and tartaric acid;
- a cosolvent in amount sufficient to solubilize the active substance;
- optionally a surfactant; and
- propellant in sufficient amount to achieve100% by weight of the finished solution.

Glucocorticosteroids having a C-20 ketone and an OH group at the C-17 and/or 21 position with varying substituents, have many well-known therapeutic uses, especially based upon their anti-inflammatory activity. This types of steroids, glucocorticosteroids, and their pharmaceutical formulations are useful in the treatment of several diseases including bronchial disorders and inflammatory conditions. Preferably, the glucocorticoid is selected between Triamcinolone Acetonide, budesonide, Dexamethasone and betamethasone 17-valerate.

A method for stabilizing aerosol pharmaceutical solution formulations containing glucocorticoids susceptible to oxidative degradation and use of a stabilizer selected between water and a mixture of water and organic acid selected between citric acid and tartaric acid are also described

## Description

The invention relates to aerosol solution formulations containing glucocorticosteroids stabilized by adding water or a mixture of water and citric acid, avoiding corrosion of the elements of container under standard storage conditions.

Particularly, the invention relates to certain 20-ketosteroids having an additional OH group in carbons 17 and/or 20 that have been found to be highly susceptible to chemical degradation when they are formulated as aerosol solution products.

### BACKGROUND OF THE INVENTION

Glucocorticosteroids having a C-20 ketone and an OH group at the C-17 and/or 21 position with varying substituents, have many well-known therapeutic uses, especially based upon their anti-inflammatory activity. These types of steroids, glucocorticosteroids, and their pharmaceutical formulations are useful in the treatment of several diseases including bronchial disorders and inflammatory conditions.

It is often desirable to deliver such steroids topically using aerosol spray devices, such as metered dose inhalers. These devices are commonly used to deliver steroids to the airways of patients via oral or by nasal inhalation for the treatment of asthma and allergic rhinitis

The aerosol medicinal products are very important as pharmaceutical dosage forms for drug administration by pulmonary route. The active substance content must remain during the shelf-life and the appearance of degradative product should be diminished so that the medicament can be used with effectiveness and security. It means, the physical and chemical stability as well as the maintenance of the quality parameters during the shelf-life of the aerosol are of essential importance for its practical medical use.

There has been a common difficulty in preparing these medicinal products delivering glucocorticoids because they are often chemically unstable formulated as aerosol formulations and degrade during storage. Therefore, their storage are inconveniently limited and expensive. Thus, the investigations have been directed vastly to avoid the degradation of steroids.

The degradation can be by oxidation and/or hydrolysis of glucocorticoids when they are in contact with the organic and inorganic solvents or by the presence of aluminum oxide contained in the material of the aerosol canister.

Therefore, the prevention of oxidative degradation may be particularly important for improving the chemical stability of glucocorticoids in solution during storage.

Chemical degradation is especially problematic when the steroid is dissolved in the formulation and, consequently most of steroid products for inhalers have been formulated as particulate suspensions of the steroid, which are much less susceptible to chemical degradation than solutions. For example, the vast majority of aerosol medicinal formulations of steroids products for their use as inhalers are available in the market only formulated as suspensions of micronized active substance in chlorofluorcarbon (CFC) propellants and hydrofluorcarbon (HFC) propellants as well.

However, this type of suspension formulations present certain disadvantages. The suspensions are heterogeneous systems. As a result of this, they are not optimal from the point of view of the uniform dosage of products nor so easily reproducible from lot to lot as solutions. In case it is possible the chemical stabilization of solutions avoiding corrosion or deterioration of the canister and valves, they continue being the best option to obtain a uniform dosage as well as an easily reproducible product from lot to lot, which is important for the industrial use of formulations.

Some steroids have recently been reformulated as solutions in non-chlorofluorcarbon (HFC) propellant with ethanol.

Moreover , some formulations have just appeared in the market, in which ones the active substance is dissolved in cosolvent and propellant mixtures. Patent U.S. 5,766,573 discloses solutions of active substances having better chemical and physical stability, such as Beclomethasone Dipropionate solutions. This document describes unexpected formulation of chemically stable solution in propellant HFC 134^{a} and/or 227 and ethanol. The patent application WO 98/52542 describes solution formulations of Ciclesonide and propellants non chlorofluorcarbons having physical and chemical stability.

Patent U.S. 5,676,930 (Jaeger et al) relates to stable aerosol solution compositions comprising medicaments that are degraded by interaction with solvents or water. Specifically, it refers to aerosol solution formulations comprising ipratropium bromide or fenoterol; ethyl alcohol; 1,1,1,2-tetrafluoroethane or 1,1,1,2,3,3,3-heptafluoropropane, and either an inorganic or an organic acid.
Jager et al, describes that the use of propellant systems containing an HFC and a cosolvent in aerosol solution formulations presents chemical stability problems. In such HFC propellant/cosolvent systems, the medicament may interact with the cosolvent and/or water which are present in the system to produce decomposition or degradation products. Thus, Jager et al teaches that adding an acid to the aerosol solution formulations provides stability against degradation or decomposition of medicament resulting from the interaction of the medicament with the cosolvent and/or the water present in the solution formulation, it means, degradation caused by hydrolysis and esterification. However, Jaeger does not teach the stabilization of a glucocorticosteroid in front of the oxidative degradation.

Moreover, in other cases, diverse alternatives have been proposed to stabilize formulations for solution aerosols, such as covering the metallic surfaces in contact with product to stabilize substances having steroid structure as it is disclosed by patent U.S. 6,315,985; or adding free radical quenchers to stabilize glucocorticoids as described in the pending patent application U.S. 20030113268.

Nevertheless, there remains a need for providing aerosol solution formulations containing glucocorticoids that resist the degradation, in particular, the oxidative degradation caused by aluminum oxide, and display improved chemical and physical stability profiles under standard conditions.

### SUMMARY OF the INVENTION

Surprisingly, the inventors of the present invention have found out interesting improvements .By adding small amounts of water or a mixture of water and organic acid, more particularly a mixture of water and citric acid, it is possible to obtain aerosol solution formulations to deliver glucocorticoids stable to the oxidative degradation induced by the present aluminum oxide in the aerosol canister under standard storage conditions.

Accordingly, it is a main objective of the present invention to provide aerosol pharmaceutical solution formulations containing a glucocorticoid as active substance and a stabilizer selected between water and a mixture of water and organic acid selected between citric acid and tartaric acid, resistant to the oxidative degradation and consequently to have better stability to the storage under standard conditions.

Another main objective of the present invention is to provide aerosol pharmaceutical solution formulations containing a stabilized glucocorticoid as active substance resistant to the oxidative degradation and consequently having good stability under standard storage conditions obtained by a simple and economic procedure.

Preferably, another objective of the present invention is to provide aerosol pharmaceutical solution formulations containing budesonide as active substance having good stability to the storage in standard conditions.

An additional objective of the present invention is to provide a method for stabilizing aerosol pharmaceutical solution formulations containing glucocorticoids, preferably budesonide.

Another additional objective of the present invention is to provide the use of water or a mixture of water and organic acid selected between citric acid and tartaric acid to stabilize aerosol pharmaceutical solution formulations containing glucocorticoids, preferably budesonide.

Therefore in accordance with the first objective of the present invention, the aerosol pharmaceutical solution formulations which are provided contain:
- a glucocorticoid having a C-20 ketone and OH group in carbons 17 and/or 21 as active substance, in a concentration between 0.05 and 1.0 % by weight;
- a stabilizer selected between water and a mixture of water and organic acid selected between citric acid and tartaric acid in an amount between 0.10 and 3 % by weight;
- a cosolvent in sufficient amount to solubilize the active substance; and
- propellant in sufficient amount to achieve 100% by weight of the finished solution.

In a preferred embodiment of the present invention, the aerosol pharmaceutical solution formulations contain:
- a glucocorticoid having a C-20 ketone and OH group in carbons 17 and/or 21 as active substance, in a concentration between 0.05 and 1.0 % by weight;
- a stabilizer selected between water and a mixture of water and organic acid selected between citric acid and tartaric acid in an amount between 0.10 and 3 % by weight;
- a cosolvent in sufficient amount to solubilize the active substance; and
- propellant in amount sufficient to achieve 100% by weight of the finished solution.

In another more preferred embodiment of the present invention, the aerosol pharmaceutical solution formulations contain:
- between 0.17 and 0.28 % by weight of a glucocorticoid having a C-20 ketone and OH group in carbons 17 and/or 21 as active substance;
- between 0.15 and 1.0 % by weight of a stabilizer selected between water and a mixture of water and an organic acid selected between citric acid and tartaric acid;
- a cosolvent in sufficient amount to solubilize the active substance; and
- propellant in sufficient amount to achieve 100% by weigh of the finished solution.

### DETAILED DESCRIPTION OF THE INVENTION

The present invention provides the stabilization of a glucocorticoid useful to obtain a solution formulation having a cosolvent and a hydrofluorocarbon propellant by adding two excipients of low cost used in inhalation therapy, such as water and an organic acid selected between citric acid and tartaric acid; without needing expensive alternatives as they are proposed in the invention claimed in patent U.S. 6,315,985. This latter involves to cover the metal surfaces in contact with the product and doing thus very difficult the industrialization or adding free radical quencher as it is taught in patent application U.S. 20030113268.

In the present invention the addition of water or a mixture of a water and an organic acid selected between citric acid and tartaric acid is proposed to stabilize a glucocorticosteroid that it is degraded by different routes, particularly by oxidation, preferably glucocorticosteroids having a C-20 ketone and OH group in carbons 17 and/or 21, as described in patent U.S. 6,315,985 in which the degradative path is catalyzed by contact with metal surfaces.

In accordance with the invention, "the glucocorticosteroid" term refers to a steroid that is either produced by the adrenal-cortex, or is chemically synthesized. The glucocorticoid of the invention includes, but not limiting, budesonide, testosterone, progesterone, estrogen, flunisolide, triamcinolone acetonide, beclomethasone (i.e., as the mono or di-propionate ester), betamethasone 17-valerate; dexamethasone, fluticasone (i.e., as the propionate ester), methylprednisolone, prednisone, hydrocortisone, ciclesonide, momethasone, desonide, and the equivalent functional.

Particularly, the glucocorticoid for the pharmaceutical formulations of the present invention is selected between the following ones: Triamcinolone Acetonide, Budesonide, Dexamethasone, Betamethasone 17-valerate.

Preferably the glucocorticosteroid is Budesonide.

The amount of glucocorticoid present in formulations of the invention is between about 0.001 and about 1 % by weight, preferably between about 0.05 and 1 % by weight; more preferably between 0.05 and 0.5 % by weight based on the total weight of the formulation. The preferred amount of glucocorticoid is between 0.17 and 0,28.

The formulations of the present invention may contain other active substances such as Formoterol fumarate and Salmeterol xinafoate.

In accordance with the present invention the formulations of the present invention are less prone to oxidative degradation. Thus, the most physically and chemically stable formulations of the invention have a longer shelf life reducing their production cost. In addition to the economic advantages, the formulations in accordance with the present invention remain stable at the range of temperatures to which these medicaments are normally exposed. One expert in the art can also easily determine the presence of oxidative degradation products, for example, by HPLC analysis. For example, a easily recognizable degradation product of budesonide is 21-dehydrobudesonide.

The addition of water improves the stability of active substances in the presence of metal oxides such as it will be demonstrated through the working examples. The preferred amount of water is approximately between 0.10 and 3 % by weight, and more preferably between 0.15 and 1 % by weight on the base of the total weight of the formulation; but it can be adjusted to each active substance.

The addition of organic acid must be done so as to achieve an acceptable stability to the active substance in contact with the definitive container. Preferably the organic acid is selected between citric acid and tartaric acid; and it is present in amounts between approximately 0.001 and 1% by weight, more preferably between 0.01 and 0.10 % by weight. Preferably the citric acid is present in an amount of approximately 0.08 % by weight.

In accordance with the present invention, the aerosols formulations are pressurized formulations that use propellants. Certain hydrofluorcarbons HFCs have suitable properties to be used like propellants for the administration by medicine aerosol. The patent application EP 89312270,5 published under number EP 372 777, describes the use of 1,1,1,2-tetrafluorethane (HFC-134(a)) in combination with at least one adjuvant and a surface active agent to prepare suspension and solution formulations of medicaments suitable for the administration by the aerosol route. Also publication WO 91/11496 discloses the use of 1,1,1,2,3,3,3-heptafluoropropane (HFC-227), optionally mixed with other propellant components for use in the preparation of aerosol suspension formulations for medicament.

Any fluoroalkane propellant that is suitable for inhalation can be used. Examples of suitable fluoroalkanes (hydrofluoroalkanes) include, without limitation, 1,1,1,2 tetrafluoroethane ("HFA-134a"), 1,1,1,2,3,3,3 heptafluoropropane ("HFA-227ea"), pentafluoroethane ("HFA-125"), 1,1-difluoroethane ("HFA-152a"), and difluoromethane ("HFA-32"). Hydrocarbon and/or aliphatic gases may be added to modify propellant characteristics. Preferably, the aerosol formulation is substantially free of chlorofluorocarbons, which have been implicated in the depletion of the ozone layer. Preferably, the fluoroalkane may be 1,1,1,2-tetrafluoroethane (HFA-134a) or 1,1,1,2,3,3,3-heptafluoropropane (HFA-227ea). The nature of the propellant used is not an essential element of the invention. Hence, other propellants can be used without affecting the formulation described in the present invention.

Usually, the propellant is present in an amount between 65 and 95% by weight on the base of total weight of the aerosol formulation

There is a great number of organic cosolvents. In accordance with the invention, by "cosolvent" is understood any cosolvent that is miscible in the formulation in the desirable amount and when is added provides a formulation in which the medicament can be dissolved in therapeutically effective amounts.

The amount of cosolvent is the effective amount capable of solubilizing the active substance. Preferably the cosolvent is in an amount between about 1 and 25%.

Examples of cosolvent that contain hydroxyl functions capable of interacting with the medicaments in the formulation can be alcohols, for example, ethyl alcohol and isopropyl alcohol; glycols for example, propylene glycol, polyethylene glycols, polypropylene glycols, glycol ethers, and other substances, such as : glycerol, polyoxyethylene alcohols, and polyoxyethylene fatty acid esters. Examples of cosolvent that may be inert to interaction with the medicament are hydrocarbons, for example, n-propane, n-butane, isobutane, n-pentane, iso-pentane, neo-pentane, and n-hexane; and ethers, for example diethyl ether.

Ethanol can be included as cosolvent to improve the solubilization of the glucocorticoid. The ethanol is a preferred cosolvent in accordance with the present invention. The ethanol is suitable since it has been used previously in aerosol formulations suitable for inhalation and it does not present toxicity problems. Other cosolvents might also be used. Some examples of alternative cosolvents are: isopropyl alcohol or propylenglycol.

The formulation can also contain surface active agents to regulate the size of drop or to improve the performance of the valve throughout the shelf life of medicines. Examples of surfactant active agents are oleic acid, sorbitan trioleate, lecithin and isopropylmiristate.

The aerosol container consists of a metal canister and a measuring dosage valve having diverse plastic (typically polyester or polyamide), metal and elastomers surfaces. The use of containers coated has been found to confer an additional protection not only the chemical stabilization of active substance but also the absence of corrosion or other unacceptable deterioration signs of the container material by contact with the product. In the present invention, the formulation in the example shows that it can stabilize chemically the active substance Budesonide, and besides it does not present corrosion signs by contact with the can or the valve.

In accordance with a second aspect of the present invention, the method of preparation of the aerosols can be made by two different ways:
1. One Step Pressure-Filling:
   - solubilizing Budesonide in the cosolvent;
   - adding organic acid selected from citric acid and tartaric acid dissolved in water to the solution;
   - transferring the solution to a reactor with hermetic zip. In case of using sole water, budesonide is dissolved in water and the solution is transferred into the reactor;
   - closing the reactor and the propellant is introduced under pressure thereto;
   - fmally, crimping the valves in the canister and then the solution present in the reactor is pressure-filled into the canisters.
2. Two Steps Filling:
   - solubilizing Budesonide in the cosolvent;
   - adding organic acid selected between citric acid and tartaric acid dissolved in water to the solution;
   - filling this solution in canister. In case of using sole water, budesonide is dissolved in water and the solution is transferred to the canisters;
   - finally crimping the valve and then the propellant is pressure-filled into the sealed canisters.

The invention will be better described in relation to the following examples, which should not be understood like a limitation of the same.

### EXAMPLE 1

The following model was adopted to illustrate the unexpected effect of the water. A solution of 150 mg of Budesonide in 50 ml of anhydrous ethanol as solvent was prepared. Then, the mixtures contained in bottle with screw tap and summarized in Table I were prepared by adding different amounts of water with and without aluminum oxide (Aldrich, Trademark, neutral, activated Brockmann I, mesh 150).

| Experiment N° | Budesonide solution (ml) | Water (µl) | Aluminum Oxide (g) |
|---|---|---|---|
| 1A | 10 | 0 | 0,000 |
| 1 B | 10 | 174 | 0,000 |
| 1C | 10 | 0 | 0,333 |
| 1D | 10 | 174 | 0,333 |

The Budesonide percentage results found after 22 hours of storage at 75°C were the following:

| Experiment N° | After 22 hours storage |
|---|---|
| 1A | 95,4 |
| 1B | 93,5 |
| 1 C | 4,9 |
| 1D | 14,7 |

It is important to clarify that this model is analogous to that adopted in patent U.S. 6,315,985 to demonstrate the negative effect of aluminum oxide on the stability of the corticoids having C-20 ketone and OH groups in carbons 17 and/or 21. Nevertheless, contrary to the assumption in that patent, the addition of water in the presence of aluminum oxide does not cause a greater diminution of the stability of Budesonide, rather a certain increase of the same one. This effect may be confirmed in example 2.

### EXAMPLE 2

Using the same model of example 1, the effect of increasing amounts of water in the stabilization of Budesonide in the presence of Aluminum oxide was investigated.

| Experiment N° | Budesonide Solution (ml) | Water (ml) | Aluminum Oxide (g) |
|---|---|---|---|
| 2.A | 10 | 0,00 | 0,333 |
| 2.B | 10 | 174 | 0,000 |
| 2.C | 10 | 870 | 0,333 |
| 2.D | 10 | 1740 | 0,333 |

The Budesonide percentage results found after 20 hours of storage at 75 °C were the following ones:

| Experiment N° | After 20 hours storage |
|---|---|
| 2.A | 17,6 |
| 2.B | 29,3 |
| 2.C | 41,6 |
| 2.D | 50,6 |

This example demonstrates clearly that the effect of adding water is favorable to the Budesonide stabilization in the presence of Aluminum Oxide and it is opposed to the assumption in patent U.S. 6,315,985 mentioned in the previous example.

### EXAMPLE 3

Based on the previous examples, aerosols containing the following formulation were prepared and tested using different commercial dispensing valves and aluminum canister without coating commonly used in aerosols inhalers.

| Component | % by weight |
|---|---|
| Budesonide | 0,1722 |
| Alcohol absolute | 10,000 |
| Water | 0,2200 |
| HFA 134a | 89,6078 |

The aerosols were storage during 6 months at 40°C and 75% of relative humidity. The total degradation percentage results were the followings:

| Type of valve | After 3 months storage | After 6 months storage |
|---|---|---|
| Valve type 1 | 2,85% | 5,20% |
| Valve type 2 | 1,63% | 2,70% |
| Valve type 3 | 3,4% | 4,2% |

The main difference between the valves is the composition of the rubber contained in the gum seals. In type 1, formulation is based on nitrile-rubber. Type 2 is based on thermoplastic elastomer and the one of type 3 is also based on thermoplastic elastomer with a later treatment.

Comparing the values described in example 8 and 9 of patent U.S. 6,315,985 that contain a similar Budesonide percentage, it can be seen that the degradation already reaches values upper to 4 % at 3 months at 40 °C and 75 % of relative humidity and, having consideration of the tendency of the displayed curve, it would surpass 8 % after 6 months in these storage conditions.

For this reason, this formulation including water and tested with the three types of valves presents clearly a greater stability; preferably, in the case of the valves of thermoplastic elastomers, the formulation fulfills the requirements of the International Norms for pharmaceutical industry (ICH Norms, International Committee for Harmonization Norms) that establish a degradation percentage inferior to 5 % after 6 months of storage at 40 °C and 75 % of relative humidity as convenient, although it is not an absolute requirement. This makes these two types commercially useful for aerosols of inhalers use. These results confirm those of examples 1 and 2 as to the stabilizing effect of the water in the degradation of Budesonide. Contrary to the hypothesis raised in patent U.S. 6,315,985, where example 6 teaches that the water presence might be unstabilized based on the results obtained from valves having disecants; the examples previously described demonstrate the stabilizing effect of the water in formulations for aerosols in accordance with the present invention. Therefore, the reached effect is unexpected front to the lessons of the previous art.

### EXAMPLE 4

Formulas with and without addition of citric acid were used but containing greater concentration of Budesonide than in example 3. The used formulas are the following ones:

| Component | Formula 4A | Formula 4B | Formula 4C | Formula 4D |
|---|---|---|---|---|
| Budesonide | 0,2834 | 0,2834 | 0,2834 | 0,2834 |
| Alcohol absolute | 17,00 | 17,00 | 17,00 | 17,00 |
| Water | 0,50 | 0,50 | 0,22 | 0,22 |
| Citric acid | --- | 0,008575 | --- | 0,0857 |
| HFA 134a | 82,22 | 82,13 | 82,50 | 82,41 |
| (content in % by weight) | | | | |

These formulations were packaged in aluminum canister commonly used in aerosols inhalers having dispensing valves of type 2 mentioned in example 3 at 40°C and 75 % of relative humidity and the following results were obtained:

| Tested formula | After 6 months storage |
|---|---|
| Formula 4.A | 2,88 |
| Formula 4.B | 8,78 |
| Formula 4.C | 8,23 |
| Formula 4.D | 5,08 |

These results unexpectedly demonstrate that the addition of water is not so effective in greater concentrations of Budesonide, however the stabilizing effect can be improved with the addition of citric acid which was never suggested in patent U.S. 6,315,985 nor in others related to the stabilization of Budesonide in formulations for aerosol inhalers. Also, based on these results it is convenient the addition of a greater amount of water to obtain greater stability of the formulation.

### EXAMPLE 5

The possible greater improvement of stability of Budesonide were analyzed when using covered canisters as suggested in patent U.S. 6,315,985. Formulation 4.A of example 4 was used.

The formulation 4.B was packaged in commonly used canister and other aluminum canisters covered with polymer and valves type 2 mentioned in example 3. These units were reversed with the valve upside down and stored during 6 months at 40°C and 75 % of relative humidity. The following results were obtained.

### Budesonide Degradation Percentage:

| Sample | After 6 months storage |
|---|---|
| Formula 4.B in common non-covered canister | 3,85% |
| Formula 4.B in covered canister | 6,31 % |

Unexpectedly, the covering of the aerosols does not produce a greater stabilization of the formulation having water and citric acid, rather the canisters without covering show an improved stability. Based on this study, it is important to emphasize that using covered canisters the recommended maximum value of 5 % of total degradation is not reached, although it is not an absolute requirement, by the International Norms for medicinal products "ICH". It is also significant that in patent U.S. 6,315,985 have not appeared data after 6 months of storage at 40°C and 75 % of relative humidity, that are so important in the medicinal product evaluation for commercial use according to International Norms "ICH" in force at least in the U.S.A., the European Community and Japan.

## Claims

1. An aerosol pharmaceutical solution formulation suitable for oral or nasal inhalation containing glucocorticoids stable to the storage, **CHARACTERIZED IN THAT** said formulation comprises:
- between 0.05 and 1.0 % by weight of a glucocorticoid having a C-20 ketone and OH group in carbons 17 and/or 21 as active substance;
- between 0.10 and 3 % by weight of a selected stabilizer selected between water or a mixture of water and organic acid selected between citric acid and tartaric acid;
- a cosolvent in sufficient amount to solubilize the active substance;
- optionally a surfactant; and
- propellant in sufficient amount to achieve 100% by weight of the finished solution.

2. A pharmaceutical formulation according to claim 1, **CHARACTERIZED by** comprising:
- between 0.05 and 0.5 % by weight of a glucocorticoid having a C-20 ketone and OH group in carbons 17 and/or 21 as active substance;
- between 0.10 and 3 % by weight of a stabilizer selected between water or a mixture of water and organic acid selected between citric acid and tartaric acid;
- a cosolvent in sufficient amount to solubilize the active substance;
- optionally a surfactant; and
- a propellant in sufficient amount to achieve 100% by weight of the finished solution.

3. A pharmaceutical formulation according to claim 1, **CHARACTERIZED by** comprising:
- between 0.17 and 0.28 % by weight of a glucocorticoid having a C-20 ketone and OH group in carbons 17 and/or 21 as active substance;
- between 0.15 and 1 % by weight of a stabilizer selected between water or a mixture of water and organic acid selected between citric acid and tartaric acid;
- a cosolvent in sufficient amount to solubilize the active substance;
- optionally a surfactant; and
- a propellant in sufficient amount to achieve 100% by weight of the final solution.

4. A pharmaceutical formulation according to any one of claims 1 to 3, **CHARACTERIZED in that** the glucocorticoid is selected from the group consisting of budesonide, testosterone, progesterone, estrogen, flunisolide, triamcinolone acetonide, beclomethasone, betamethasone 17-valerate; dexamethasone, fluticasone, methylprednisolone, prednisone, hydrocortisone, ciclesonide and momethasone.

5. A pharmaceutical formulation according to any one of claims 1 to 4, **CHARACTERIZED in that** the glucocorticoid is selected between triamcinolone acetonide, budesonide, dexamethasone and betamethasone 17-valerate.

6. A pharmaceutical formulation according to any one of claims 1 to 5, **CHARACTERIZED by** further comprising other active substances as for example Formoterol fumarate and Salmeterol xinafoate.

7. A pharmaceutical formulation according to any one of claims 1 to 5, **CHARACTERIZED in that** the glucocorticoid is budesonide.

8. A pharmaceutical formulation according to any one of claims 1 to 5, **CHARACTERIZED in that** the stabilizer is water in an amount of 0, 22 % by weight of the finished formulation.

9. A pharmaceutical formulation according to any one of claims 1 to 5, **CHARACTERIZED in that** the stabilizer is a mixture of water in an amount between 0,20 and 0,50% by weight and citric acid in an amount of about 0,085% by weight of the total formulation.

10. A method for stabilizing aerosol pharmaceutical solution formulations containing glucocorticoids susceptible to oxidative degradation, **CHARACTERIZED in that** said method comprises:
- solubilizing the active substance, a glucocorticoid having a C-20 ketone and OH group in carbons 17 and/or 21, in an effective amount of cos lvent; and
- adding a stabilizer selected between water and a mixture of water and organic acid selected between citric acid and tartaric acid.

11. A method according to claim 10, **CHARACTERIZED in that** the glucocorticoid is budesonide.

12. Use of a stabilizer selected between water and a mixture of water and organic acid selected between citric acid and tartaric acid **CHARACTERIZED in that** it is for stabilizing aerosol pharmaceutical solution formulations containing glucocorticoids having a C-20 ketone and OH group in carbons 17 and/or 21 and susceptible to oxidative degradation.

13. The use according to any one of claim 12, **CHARACTERIZED in that** the glucocorticoid is budesonide.
